# EUROPEAN PATENT SPECIFICATION

(11) **EP 1 339 428 B1**
(45) Date of publication and mention of the grant of the patent: **21.02.2007**
(21) Application number: 01983064.5
(22) Date of filing: 15.11.2001
(51) Int. Cl.: A61K 45/06, A61K 31/196, A61K 33/00, A61P 11/08, A61P 11/06

(54) **INHALATION OF NITRIC OXIDE**
INHALATION VON STICKOXID
INHALATION D'OXYDE NITRIQUE

(30) Priority: 17.11.2000 SE 0004229
(43) Date of publication of application: 03.09.2003
(73) Proprietor: AGA AB, 181 81 Lidingö (SE)
(72) Inventor: HEDENSTIERNA, Göran, S-182 64 Djursholm (SE); CHEN, Luni, S-756 46 Uppsala (SE)
(74) Representative: Larsson, Kjell
(86) International application number: PCT/SE2001/002542
(87) International publication number: WO 2002/040052

(56) References cited:
- WO-A1-92/10228
- US-A- 4 536 515
- US-A- 4 537 906
- H.L. LIPPTON ET AL.: 'Influence of cyclooxygenase blockade on responses to isoproterenol, bradykinin and nitroglycerin in the feline pulmonary vascular bed' PROSTAGLANDINS vol. 28, no. 2, August 1984, pages 253 - 270, XP002908109
- JUNKO MAUYAMA ET AL.: 'Impaired nitric oxide-dependent responses and their recovery in hypertensive pulmonary arteries of rats' AM. J. PHYSIOL. vol. 266, 1994, pages H2476 - H2488, XP002908110

## Description

### Field of the invention

The present invention is within the field of medicaments for treatment of pulmonary vasoconstriction or airway constriction in a mammal, especially man. The invention is intended for the treatment of individuals who are the subject of a rebound response on discontinuation of nitric oxide inhalation.

### Background of the invention

Nitric oxide relaxes pulmonary vessels, in particular when they are constricted by various disorders, as will be exemplified below. Nitric oxide also relaxes airway smooth muscle (Belvisi MG, Stretton CD, Barnes PJ. Eur. J. Pharmacol. 1992; 210: 221-222), and inhalation of exogenous nitric oxide attenuates airway constriction in the response to various agents in laboratory animals and humans (Dupuy PM, Shore SA, Drazen JM, Frostell C, Hill WA, Zapol WM. J.Clin.Invest. 1992; 90:421-428; Högman M, Frostell C, Arnberg H, Hedenstierna G. Eur.Respir.J. 1993; 6:177-180; Högman M, Frostell CG, Hedenström H, Hedenstierna G. Am.Rev.Respir.Dis. 1993; 148:1474-1478). Thus, for instance EP 560 928, US 5,485,827, 5,873,359 and WO 92/10228 disclose the use of nitric oxide for treating bronchoconstriction and pulmonary vasoconstriction. It has however been found that the effect of the treatment shows great inter- and intra- individual variability. Furthermore, although NO inhalation (INO) may be an efficient therapy in patients with pulmonary hypertension, around 1/3 of the patients are hypo-or non-responders to INO. In addition thereto, worsening of the pulmonary hypertension and of the oxygenation have been observed during attempts to withdraw INO, which is termed rebound response. Life-threatening hemodynamic instability and deaths by discontinuing inhalation of nitric oxide have also been reported. Stepwise lowering of the NO dose will prolong the NO therapy but may still not eliminate the rebound response.

The mechanisms responsible for the hyporesponsiveness and the rebound response are not fully understood. One of the hypothesisis is that the endogenous NO production can be inhibited by NO inhalation as a negative feedback mechanism with down-regulation of the endogenous synthesis. In a study forming the basis for the present invention an animal model was developed which produced a rebound response on discontinuation of NO inhalation by endotoxin infusion for at least 3 hours. Moreover, the findings indicated that the rebound was not only caused by a down-regulation of endogenous NO production but also, and possibly more important, by increased activity of the vasoconstrictor Endothelin 1 (ET-1). Another observation was that there was an inverse correlation' between the response to INO and the degree of rebound. Thus, the poorer the effect of INO, the stronger was the rebound response. A similar link between hypo- or non-response and rebound has been found by Davidson and coworkers in neonatal respiratory distress cases. (D. Davidson, MD, Pediatries. 104(2): 231-236, 1999).

In our model, endotoxin infusion caused a biphasic increase in pulmonary artery pressure and decrease in PaO2. An initial, severe increase in the pulmonary hypertension appeared 15-30 minutes after onset of endotoxin infusion in parallel to increased concentrations of the vasoconstrictor Thromboxane A2 (TXA2) or other cyclooxygenase (COX) products. A second, stable, period of pulmonary hypertension and hypoxia appeared 2,5 hours after onset of endotoxin infusion, in parallel with an increased ET-1.

Several studies have reported that INO failed to reverse the pulmonary vasoconstriction induced by a thromboxane analogue (Welte M et al., Acta Physiol Scand 1995, July, 154(3): 395-405). Ikeda S et al. (Ikeda S., Shirai M., Shimouchi A., Min KY., Ohsawa N., Ninomiya I., J.Physiology 1999 Feb; 49(1): 89-98) reported that INO combined with intravenously administered prostacyclin can produce a more enhanced vasodilator effect.

Furthermore, specific substituted phenylalkenoic acid and esters which inhibit bronchoconstriction induced by leukotrienes or arachidonic acid are known per se from US 4,536,515 and US 4,537,906.

Finally, Lippton H.L. et al; "Influence of cyclooxygenase blockade on a response to isoproterenol, bradykinin and nitroglycerin in the feline pulmonary vascular bed"; Prostaglandins; 1984, Vol. 28. No. 2, pp. 253-270, disclose that cyclooxygenase products such as PGI₂ do not mediate vasodilator response to isoproterenol, bradykinin and nitroglycerin in the pulmonary vascular bed.

Based on the above-mentioned it is highly surprising that a combination therapy for an effective tretament of pulmonary vasoconstriction or airway constriction, i.e. where the side-effects of INO as described above can be eliminated or at least extensively reduced, can be achieved in accordance with the present invention.

### Summary of the invention

Accordingly the object of the invention is to provide suitable compounds for use to counteract or eliminate a rebound response when discontinuing inhalation of nitric oxide.

A further object of the invention is to accomplish the use of a pure inhalable medicament.

The above-mentioned object are accomplished by the use, method and pharmaceutical preparation defined in the accompanying claims.

According to the invention there is provided a use of inhalable nitric oxide(NO) in combination with a cyclogenase (COX) inhibitor for the manufacture of a medicament to counteract a rebound response in the case of withdrawal of treatment with gaseous nitric oxide-or nitric oxide donor only, said combination being used in a therapeutically effective amount to accomplish said counteraction.

Thus, according to the present invention it was surprisingly found that the reduced relaxing effect of exogenous nitric oxide, or even life-threatening effect when discontinuing inhalation of nitric oxide, is, at least partly, due to cyclooxygenase products.

More specifically, it has been found that when a cyclooxygenase (COX) inhibitor is used in combination with nitric oxide, the rebound response appearing when discontinuing nitric oxide inhalation is attenuated or even eliminated.

### Detailed description of the invention

The combination of gaseous nitric oxide and COX inhibitor according to the present invention can be used for the manufacture of a medicament for treating a rebound response appearing at the discontinuation of NO inhalation.

According to the invention nitric oxide is preferably used in gaseous, inhalable form. Inhalation of gaseous nitric oxide represents a great advantage in therapy, e.g. in comparison with a non-gaseous nitric oxide-donor, as the gas has no particles or droplets to disperse and transport to the respiratory tract. Gases have long free-diffusion pathways, more easily bypass obstructions (such as constricted airways) than particles or droplets, and dissolve directly in tissue without causing impaction bronchospasm. The beneficial effect of NO gas on pulmonary vascular and airway smooth muscle tone is observed immediately following inhalation, making NO a useful first defense against bronchospasm as well as pulmonary vasoconstriction that can be followed, if desired, by inhalation of longer-acting agents.

However, according to another embodiment the nitric oxide is administered in the form of a nitric oxide donor, i.e. a compound that act by releasing nitric oxide. Known nitric oxide releasing compounds useful in practice of the invention are nitroso or nitrosyl compounds such as S-nitroso-N-acetylpenicillamine, S-nitroso-L-cysteine and nitrosoguanidine,characterized by an -NO moitey that is spontaneously released or otherwise transferred from the compound under physiological conditions such as obtained in the lung. Other compounds are compounds in which NO is a ligand on a transition metal complex and as such is readily released or transferred from the compound under physiological conditions, e.g. nitroprusside, NO-ferredoxin, or an NO- heme complex. Further suitable nitrogen-containing compounds are compounds which are metabolized by enzymes endogenous to the respiratory and/or vascular system to produce the NO radical, e.g. arginine, glycerol trinitrate, isoamylnitrite, inorganic nitrite, azide and hydroxylamine. Such types of nitric oxide releasing compounds and method for their synthesis are well known in the art. Preferably the nitric oxide donor is a compound that releases nitric oxide in such a way that only the airways and the pulmonary vessels are affected.

The nitric oxide donor used in the invention may be administered as a powder(i.e. finely divided solid, either provided pure or as a mixture with a biologically compatible carrier powder, or with one or more additional therapeutic compounds) or as a liquid(i.e. dissolved or suspended in a biologically compatible liquid carrier, optionally mixed with one or more additional therapeutic compounds), and can conveniently be inhaled in nebulized form (preferably including particles or droplets having a diameter of less than 10 µm). Carrier liquids and powders that are suitable for inhalation are commonly used in traditional asthma inhalation therapeutics and thus well known in the art. The optimal dosage range can be determined by routine procedures known to the skilled man.

The cyclooxygenase inhibitor to be used in the invention can be any compound recognized as being suitable to mammalian, especially human, use, which can be conveniently administered. The experiments performed in connection with the present invention are experiments with an unselective COX-inhibitor. However, blockage of the COX-2 enzyme appears to augment and prolong the effect of INO. Blockage of the COX-1 and COX-2 enzymes may both blunt the rebound response. The use of an unselective COX-blocker should therefore be useful, but advantageous effects may be acheived with a more selective COX-blocker as well.

However, some examples of COX inhibitors which should work in accordance with the present invention are: diclofenac; aceclofenac; nabumetone; meloxicam; meclofenamic; nimesulide; paracetamol; rofecoxib; celecoxib; DuP 697 (5-bromo-2-(4-fluorophenyl)-3-[4-(methylsulfonyl)-phenyl]thiophene); GR 32191 (((IR-α(Z), 2β, 3β, 5α))-(+)-7-5(((1.1'-biphenyl)-4-yl)-methoxy)-3-hydroxy-2-(1-piperidinyl)-cyclopentyl)-4-heptenoic acid); flosulide(or cGP 28238); NS 398 (N-(2-(cyclohexyloxy)-4-nitrophenyl)-methansulfonamide); L-745,337(N-[6-[(2,4-difluorophenyl)thio]-2,3-dihydro-1-oxo-1H-inden-5-yl]methanesulfonamide); DFU((5,5-dimethyl-3-(3-fluorphenyl)-4-(4-methylsulfonyl)phenyl-2(5H)-furanone); HN-56249((3-2,4-dichlorothiophenoxy)-4-methylsulfonylamino-benzenesulfonamide); JTE-522((4-(4-cyclohexyl-2-methyloxazol-5-yl)-2-fluorobenzenesulfonamid); aspirin; indometacin; and ibuprofen.

Of these specific compounds aspirin, indometacin and ibuprofen appear to be more selective relative to COX-1 inhibiton, while the other compounds (at least most of them) appear to be more selective relative to COX-1 and COX-2 inhibition or essentially similarly selective relative to COX-2 inhibition. Also acid addition salts thereof, e.g. hydrochlorides, may be useful.

The components used according to the invention can be administered by, commercially available, inhaler devices. Compressed NO gas may be obtained from a commercial supplier, typically as a mixture of 200-2000 ppm NO in pure N₂ gas. Said NO-N₂ gas mixture may be delivered into the inhalation gas in an amount of 1 - 100000 nmol/min or, may be mixed with air, oxygen or another suitable carrier gas or gas mixture, generally to a concentration of 1 ppm to 180 ppm of said mixture. For inhalations during extended periods of time a range of 1-40 ppm is generally utilized, while 1-80 ppm or 1-180 ppm may be used for shorter periods of time, when an immediate strong effect is desired. Especially preferable ranges in the last-mentioned cases are 20-80 ppm (e.g. 40-80 ppm) or 40-180 ppm, respectively. As to further details concerning inhalation of NO reference is made to the prior art, e.g. EP 560 928 B1, in this respect.

The nitric oxide and the cyclooxygenase inhibitor can be administered sequentially in any order, or they can be administered simultaneously, in the latter case either with the two components from separate sources at the same time or together, or, in the form of a composition comprising both said nitric oxide and said cyclooxygenase inhibitor.

The cyclooxygenase inhibitor can be administered in the same manner as NO, i.e. by inhalation, but also by other common administration routes for pharmaceuticals. Among such routes reference can be made to sublingual, oral, and rectal administrations, application to epithelial surfaces, and injection, which may be subcutaneous, intramuscular, intravenous or intraperitoneal. Preferably, however, the cyclooxygenase inhibitor is administered by inhalation. Thus it may be administered as a powder(i.e. finely divided solid, either provided pure or as a mixture with a biologically compatible carrier powder, or with one or more additional therapeutic compounds) or as a liquid(i.e. dissolved or suspended in a biologically compatible liquid carrier, optionally mixed with one or more additional therapeutic compounds), but can conveniently be inhaled in nebulized form (preferably including particles or droplets having a diameter of less than 10 µm). Carrier liquids and powders that are suitable for inhalation are commonly used in traditional asthma inhalation therapeutics and thus well known in the art.

The cyclooxygenase inhibitor is used in a therapeutically effective amount, such an amount being easily established by the skilled artisan, dependent inter alia on the type of compound used and the route of administration. As should be apparent to a person skilled in the art the term "therapeutic" in this respect as well as in general in the description and claims encompasses prophylactic treatment as well as treatment of an established condition. Furthermore, "therapeutically effective" is utilized in a sense that is common within this technical field, as is e.g. defined in EP 560 928 B1 referred to above, although in general in this specific case the cyclooxygenase inhibitor is therapeutically effective as soon as it reverses a negative effect obtained when using gaseous nitric oxide only. As a guidance in this respect it could also be added that the compound diclofenac is generally effective when used in a total amount of 50-150 mg/day for an adult when used by oral tablets, suppositories, intramuscular injection or intravenous infusion. This should correspond to a dose of approximately 0.1 to 5 mg/kg body weight, such as 0.15 to 3 mg/kg body weight, when used in the form of an aerosol. However, effects might sometimes be obtained at a lower dose and sometimes even higher doses might be required. For other compounds this could be used as a basis for the establishment of appropriate dosages thereof.

A method is described for the treatment of pulmonary vasoconstriction or airway construction in a mammal, especially man in order to counteract a hypo- or non-response to treatment with gaseous nitric oxide or nitric oxide donor only and/or to counteract a rebound response in the case of withdrawal of treatment with gaseous nitric oxide or nitric oxide donor only. Said method comprises administering by inhalation, to a mammal in need of such treatment, nitric oxide (NO), in the form of gaseous nitric oxide or a nitric oxide donor, in combination with a cyclooxygenase inhibitor, said combination being used in a therapeutically effective amount to accomplish said counteraction.

As to specific and preferable embodiments of said method reference is made to those specific and preferable embodiments which have been described in connection with the use according to the invention.

Finally, there is described a pharmaceutical preparation for treatment of pulmonary vasoconstriction or airway constriction in a mammal, especially man, in order to counteract a hypo- or non-response to treatment with gaseous nitric oxide or nitric oxide donor only and/or no counteract a rebound response in the case of withdrawal of treatment with gaseous nitric oxide or nitric oxide donor only which comprises nitric oxide (NO), in the form of gaseous nitric oxide or a nitric oxide donor, in combination with a cyclooxygenase inhibitor, said gaseous nitric oxide and said being present in a therapeutically effective amount to accomplish said counteraction.

With reference to specific and preferable embodiments of said preparation reference is also made to said specific and preferable embodiments of the use according to the invention.

The invention will now be illustrated by the following non-limiting examples:

### Example

Experiments were performed to investigate the effect of a COX inhibitor, diclofenac, in connection with inhalation of NO, more specifically on the rebound response on discontinuation of said inhalation.

### Materials and methods

### Animal preparation

The Animal Research Ethics Committee of Uppsala University approved the study. Twentysix pigs of Swedish country breed, weighing 24-29 Kg, were used in the study. Before the transport from the farm, the pigs were sedated with a neuroleptic, azaperonum (STRESNIL, Janssen, Belgium), 40mg intramuscularly (im). Anaesthesia was induced with atropine im (0.04 mg/Kg), tiletamin/zolazepam (ZOLETIL, Virbac Laboratories) (6 mg/Kg) and xylzry (ROMPUN, Bayer AG, Germany) (2.2 mg/Kg). After induction, a cannula was inserted in an ear vein and an opioid (FENTANYL, Antigen Pharmaceuticals Ltd, Roscrea, Ireland) (5ug/Kg) was injected. Muscle relaxation was provided by pancuronium (PAVULON, Organon Technika AB, Göteborg, Sweden) (0.2 ug/kg). Anaesthesia was maintained with a continuous infusion of a hypnotic (chlomethiazole, HEMINEVRIN, Astra, Södertälje, Sweden) (400mg /hour), pancuronium (2mg/hour) and fentanyl (150 ug/hour). Repeated doses of fentanyl 0.2-0.5 mg i.v. were given as necessary. Depth of anaesthesia was considered to be adequate when skin incisions did not cause any changes in heart rate or blood pressure. Pre-warmed (38°C) isotonic saline 500 ml/ hour was given for hydration. The animals were placed in the supine position (dorsal recumbency) for the remainder of the study.

A tracheotomy was performed after induction of anaesthesia and a cuffed tracheal tube (inner diameter 6mm) was inserted. A volume-controlled ventilator (Siemens 900c) provided mechanical ventilation. Transducers built into the ventilator recorded airway pressure and minute ventilation. Respiratory frequency was maintained at 20 breaths per minute with tidal volume adjusted to maintain an end-tidal CO2 (PetCO2) between 36 and 41 mm Hg (4.8-5.4 kPa). The inspiratory time was 25%; an end-inspiratory pause 5% of the respiratory cycle was applied, as well as an end-expiratory pressure (PEEP) of 5-cm H2O. Inspired fraction of oxygen (FI02) was 0.5.

### Catheterizations and blood measurements

A triple lumen balloon-tipped catheter (Swan Ganz no.7F) was introduced via the right external jugular vein to the pulmonary artery for blood sampling and pressure recordings. A large bore catheter was inserted into the contralateral jugular vein for infusion purposes, with its tip in the superior caval vein. The right carotid artery was cannulated for blood sampling and recording of arterial blood pressure.

The arterial, central venous and pulmonary artery catheters were connected to appropriate pressure transducers (Sorenson Transpac Transducers, Abbott Critical Care Systems, Ill, USA) and pressures were recorded on a Marquette 7010 monitor (Marquette Electronics Inc., WI USA). Mean arterial pressure (MAP), mean pulmonary artery pressure (MPAP), heart rate (HR), central venous pressure (CVP), pulmonary capillary wedge pressure (PCWP) and cardiac output (Qt) were recorded. Airway pressures were registered from the ventilator. Vascular pressures were averaged over the whole respiratory cycle and the mid-thorax was used as the zero reference level.

Qt was measured by means of thermodilution: 10 ml of ice cold isotonic saline were injected as a bolus and the Qt was computed (cardiac output computer Marquette 7010, Marquette Electronics Inc., WI, USA). At least three injections were given for each measurement and the mean was calculated. The injections were evenly distributed over the respiratory cycle. The expired minute volume was recorded at each measurement. Mixed venous and arterial blood samples were collected for blood gas analysis (ABL 3, Radiometer, Copenhagen, Denmark), oxygen saturation and hemoglobin concentration (OSM 3, Radiometer, Copenhagen, Denmark). Five-ml arterial blood were collected at the same time, the plasma was separated for biochemical analysis (see below).

### Lung injury

Acute lung damage was induced by intravenous infusion of endotoxin 25 ug/kg/hour for 3 hours, and then maintained at 10 ug/kg/hour during the remaining experimental period.

### Protocol

Thirty minutes after surgery, baseline measurements of hemodynamics were made, blood samples were taken and blood was collected for subsequent biochemical analysis. Acute lung injury was created by an intravenous infusion of endotoxin (LPS, E Coli 0111:B4, Sigma Chemicals, St Louis, Mo, USA) 25 ug/kg/hour for 3 hours. The response was measured 30, 60, 120, and 150 minutes after the onset of the endotoxin infusion. The animals were then divided into three groups in order to analyze the potential INO-boosting effect of a COX inhibitor. This was achieved by allocating the pigs to", a "Cyclooxygenase (COX) blockage group" a "no-block" group, and a "control group".
1. In the COX blockage group, the endotoxin infusion lasted for three hours before the inhalation of 30 ppm of INO was started. A non-selective COX inhibitor Diclofenac (Sigma D6899) in saline (300 mg/Kg) was given as an iv bolus 30 minutes before NO inhalation. Before the discontinuation of INO 30 minutes later, measurements of hemodynamics and gas exchange were made and blood was sampled for biochemistry analysis. Measurements were also made when the NO had been withdrawn for 5, 10, 15, 30 minutes to check for evidence of a rebound reaction. NO inhalation was then given for another half-hour (i.e. four hours after onset of endotoxin infusion) and withdrawn again. The results were studied at the same time points as before. This was done in order to see if the response to INO remained or had changed and if any rebound response was stronger than, or had appeared after the first occasion.
2. In the no-block group, no COX inhibitor was given. The protocol was otherwise similar to that used in the COX blockage group. In 5 pigs lung tissue samples were taken via a sternotomy at 4 occasions (before, during and after NO inhalation) during the study.
3. The control group was given the same infusion of endotoxin but was not challenged with INO or a COX inhibitor. The control pigs were studied at baseline and at time points coinciding with the start and end of the two INO challenges, and at the end of the experiment, five hours after commencement of the endotoxin administration.

The study period after commencement of endotoxin infusion was thus 300 minutes (five hours) and the total study time, including anesthesia, preparation and baseline measurement before endotoxin, was approximately 7 hours.

### NO administration and recording of NO in respiratory air

NO, 1000 ppm in N2 was mixed with a blend of 02/N2 and connected to the low-flow inlet of the ventilator. The inspired gas passed through a canister containing soda lime to absorb any NO2. The concentrations of the inspired NO and NO2 were measured by chemiluminiscence (9841 NOx, Lear Siegler Measurement Controls Corporation, Englewood CO, USA) in the inspiratory limb of the ventilation tubing. Inspired NO was set to 30 ppm and inspired NO2 was always less than 0.5 ppm.

### COX-1 and COX-2 expression by Western blotting

The lung tissue blocks were rinsed by PBS at 4°C. The total protein of lung tissue was extracted by homogenization (Ultra-Turrax, Jenke and Kunkel, IKA Labortechnik, Staufen, Germany) in 5 volumes of ice-cold 0.05M Tris buffer (pH 7.4) containing 0.5 mM phenylmethyl-sulfonylfluoride to inhibit proteolysis. The supernatant was collected and stored at -80°C until the analysis. The concentration of whole protein in the supernatant was determined by the method of Lowry. Then the protein was fractionated by sodium dodecyl sulfate-polyacrylamide gel electrophoresis, and electrophoretically transferred to a nitrocellulose membrane. The blot was blocked with 5 % BSA in TBS overnight at 4°C and then incubated with anti-COX-1 (1:2500, Cayman Chemical, MI, USA, Ca 160108), COX-2 (1:1500, Cayman Chemical, MI, USA, Ca 160108) in TBS containing 1 % BSA over night at 4°C. After washing with TBS five times, the blots were incubated for 1 hour with horseradish peroxidase-conjugated goat anti-rabbit immunoglobulin G (Ig G) (1:2500 dilution, Vector Laboratories, Burlingame, USA) for ET-1 detection. The blot was washed 5 times in TBS,and the antigen-antibody complex was detected on photographic film, using enhanced chemiluminescence reagent (Amersham, Arlington Heights, IL, USA). All the experiments were carried out three times, and the bands from each experiment were analyzed using the program of National Institutes of Health (NIH) Image 1.6 C for statistical analysis.

### Plasma TXB2

Blood was collected in pre-chilled tubes containing EDTA (10 mM, final concentration), centrifuged (10 min 40C). Measurements of TXB2 concentration were performed using a commercially available enzyme immunoassay (Thromboxane B2 EIA kit Cayman Chemical, MI, USA, Ca 519031). Serum was collected from each test tube and stored at -70 ° C until TXB2 measurements were performed. To ensure that all samples were free of organic solvents, the serum was purified before its addition to the assay well. Purification of TXB2 was performed as the Cayman Chemical Introduction, by first adding 10,000 cpm of tritium-labeled TXB2 (3H -TXB2) to each sample, and then 2 ml ethanol, followed by votex 4 °C for 5 minutes, and centrifugation at 1500 g for 10 minutes to remove precipitated proteins. The supernatant was collected and mixed with ultra-pure water. The sample was passed through a C-18 reverse phase cartridge. Ten percent of the eluate was removed for scintillation counting.

Enzyme immunoassays were performed in duplicate by mixing 50 µl purified sample with 50µl tracer and 50 µl antiserum on micro-plates. After 18 hours incubation, 200 µl Ellmans-Reagents was added to start the enzymatic reaction, and the absorption of individual vials was measured 30 minutes later at 405 nm using a photometry micro-plate reader (Thermo Max, Molecular Devices ). Concentrations of TXB2 in the samples were estimated from standard curves obtained by nonlinear regression to absorption of eight known TXB2 concentrations ranging from 7.5 to 1000 pg/ml. The intra-assay and inter-assay coefficients of variation were < 10 %. For further details, see the TXB2 Enzyme immunoassay Kit 519031 Cayman Chemical manual.

### Statistical analysis

Mean and standard deviation of the mean was calculated for all variables under all study conditions. Comparisons were made using analysis of variance (ANOVA) for repeated measurements; corrections were made for multiple comparisons. Differences were considered significant at a level of p < 0.05.

### Results

### Endotoxin induced lung damage

Baseline hemodynamics and arterial oxygenation were similar to those measured in healthy pigs in previous experiments (Freden F. et al., Br J Anaesth, 77(3):413-8, 1996). There were no differences between the three groups

Endotoxin infusion increased MPAP more than two-fold after 150 min of infusion in all groups. PaO2 was significantly reduced, to half the baseline value. In addition, heart rate increased whereas cardiac output decreased. There were no significant differences between measurements made at 3 and 4 hours after commencement of the endotoxin infusion in the UN-COX group, and after 3, 4 and 5 hours in the control group.

### NO inhalation and discontinuation

In the no-block group, after 3 hours of endotoxin infusion, inhalation of 30 ppm NO resulted in a 28% decrease in MPAP and a 54% increase in PaO2. When, after 30 minutes, the NO inhalation was discontinued, MPAP rapidly increased (within 5 minutes) to a level that was 23% (9 mmHg) higher than before the NO inhalation; thus a rebound response had developed. PaO2 decreased rapidly to the pre-NO inhalation value but did not show a clear rebound phenomenon.

When the NO inhalation was repeated after 4 hours of endotoxin infusion, the fall in MPAP and increase in PaO2 were weaker, and no longer significant for PaO2, than during the test one hour earlier. Five minutes after the discontinuation of the NO inhalation, MPAP had again increased significantly to above the pre-NO level (+26%) and PaO2 had fallen to below the pre-NO level. Thus, a clear rebound hypoxemia was seen.

There was an inverse relationship between the improvement in PaO2 on NO inhalation and the magnitude of the rebound response on discontinuation of NO. Thus, the weaker the response to inhaled NO, the greater the fall in PaO2 when NO was discontinued. No such relationship was seen for MPAP.

In the COX-blockage group, pretreatment with Diclofenac lowered MPAP. INO significantly decreased MPAP and increased PaO2. However, on discontinuation of INO, no rebound was seen in either MPAP or PaO2. The magnitude of the PAP reduction was not greater by the combination of INO and diclofenac. However it prolonged the duration of action of inhaled NO compared with the no-block group. Moreover, INO produced the same improvement in MPAP and PaO2 in the second trial of NO inhalation as during the first trial one hour before, contrary to the attenuated response to NO in the no-block group. Again no rebound response was recorded when INO was discontinued. The respiratory resistance increased during the endotoxin infusion. INO alone did not prevent or attenuate the rise. However, in the COX-blockage group the increase in resistance was significantly lower (p<0.01) and there was no rise at all during the INO periods.
In the control group, MPAP and PaO2 remained stable over the two hours, corresponding to the INO challenges in the study groups. Thus, a comparison between the INO and control groups disclosed even more clearly the improvements in MPAP and PaO2 by the two INO-challenges, the rebound response on discontinuation of INO and the effects of COX inhibitor.

The inhalation or the discontinuation of NO did not affect the COX-1 expression, neither at the 3 nor at the 5 hour INO trial. The COX-2 expression was markedly increased after 3 hours of endotoxin infusion in the no-block group with a further increase at 5 hours to almost 10 times normal baseline levels (p<0.01). Thirty minutes of INO had no effect on the expression, neither during the 3 nor during the 5 hour inhalation test. Also, the COX-2 expression remained increased after the INO periods.

### Plasma TXB-2 concentration:

There was no significant difference at base line between the three groups. The plasma TXA2 was dramatically increased (6000- 80000 pg/ml) half an hour after onset of endotoxin infusion and was then decreased to twice the baseline at 3 hours, with no difference between the groups. In the no-block group, the plasma TXB2 level did not change during NO inhalation, but was increased five minutes after INO withdrawal and peaked at 15 min after INO (p< 0.05). It remained elevated when the second INO trial was commenced 30 minutes later ( 20000 - 40000 pg/ml) with a further increase when the INO was withdrawn for the second time. In the COX-blockage group, the plasma TXA2 did not increase by the first INO challenge and withdrawal. There was a decline of the plasma TXB2 during the second INO trial and there was no increase after the INO withdrawal.

## Claims

1. Use of inhalable nitric oxide (NO), in the form of gaseous nitric oxide or a nitric oxide donor, in combination with a cyclooxygenase inhibitor for the manufacture of a medicament for treating pulmonary vasoconstriction or airway constriction in a mammal, especially man, in order to counteract a hypo- or non-response to treatment with gaseous nitric oxide or nitric oxide donor only and/or to counteract a rebound response in the case of withdrawal of treatment with gaseous nitric oxide or nitric oxide donor only

2. Use according to claim 1, wherein said pulmonary vasoconstriction or airway constriction is associated with a clinical condition selected from the group consisting of traumatic injury, fat embolism in the lung, acidosis, adult respiratory distress syndrome, acute mountain sickness, post cardiovascular and pulmonary surgery acute pulmonary hypertension, persistent pulmonary hypertension of the new-born, perinatal aspiration syndrome, hyaline membrane disease, acute pulmonary thromboembolism, acute pulmonary thromboembolism, acute pulmonary edema, heparin-protamine reactions, hypoxia and asthma bronchiale.

3. Use according to claim 2, wherein said airway constriction is associated with asthma bronchiale.

4. Use according to claim 3, wherein said airway constriction is associated with an acute condition of asthma bronchiale or status asthmaticus.

5. Use according to any one of the preceding claims, wherein said medicament is an inhalable medicament.

6. Use according to any one of the preceding claims, wherein said manufacture relates to a medicament for sequential administration of said nitric oxide and said COX inhibitor in any order.

7. Use according to any one of claims 1-5, wherein said medicament is in the form of a composition comprising said nitric oxide and said cyclooxygenase inhibitor for simultaneous administration thereof.

8. Use according to any one of the preceding claims, wherein said cyclooxygenase inhibitor is selected from the group consisting of diclofenac; aceclofenac; nabumetone; meloxicam; meclofenamic; nimesulide; paracetamol; rofecoxib, celecoxib, DuP 697; GR 32191; flosulide; NS 398; L-745,337; DFU; HN-56249; JTE-552; aspirin; indometacin; and ibuprofen; or acid addition salts thereof.

9. Use according to any one of the preceding claims, wherein NO is present in said medicament in an amount of 1-100 nmol/min.

10. Use according to any one of the preceding claims, wherein the concentration of NO to be inhaled is within the range of 1-180 ppm, preferably 1-80 ppm, especially 1-40 ppm, said NO being present in a carrier gas or gas mixture.

11. Use according to any one of the preceding claims, wherein said nitric oxide donor is selected from S-nitroso-N-acetylpenicillamine, S-nitrosocysteine, nitroprusside, nitrosoguanidine, glycerol trinitrate, isoamylnitrite, inorganic nitrite, azide and hydroxylamine.

## Patentansprüche

1. Verwendung von inhalierbarem Stickoxid (NO) in Form von gasförmigem Stickoxid oder einem Stickoxiddonor in Kombination mit einem Cyclooxigenaseinhibitor für die Herstellung eines Medikamentes zum Behandeln einer Verengung der Lungengefäße oder einer Verengung der Atemwege bei einem Säugetier, insbesondere beim Menschen, um einer Unterreaktion oder nicht vorhandenen Reaktion auf eine alleinige Behandlung mit gasförmigem Stickoxid oder einem Stickoxiddonor entgegen zu wirken und/oder einem Rezidiv im Fall des Absetzens der alleinigen Behandlung mit gasförmigem Stickoxid oder einem Stickoxiddonor entgegen zu wirken.

2. Verwendung nach Anspruch 1, wobei die Verengung der Lungengefäße oder die Verengung der Atemwege mit einem klinischen Zustand assoziiert ist, der aus der Gruppe ausgewählt ist, die aus einer traumatisch bedingten Verletzung, einer Fettembolie in der Lunge, Azidose, Atemnotsyndrom bei Erwachsenen, akuter Höhenkrankheit, akutem Lungenhochdruck nach einer Herz- oder Lungenoperation, persistierendem Lungenhochdruck bei Neugeborenen, perinatalem Aspirationssyndrom, Surfactantmangelsyndrom, akuter Lungenthromboembolie, akutem Lungenödem, Heparin-Protamin-Reaktionen, Hypoxie und Asthma bronchiale besteht.

3. Verwendung nach Anspruch 2, wobei die Verengung der Atemwege mit Asthma bronchiale assoziiert ist.

4. Verwendung nach Anspruch 3, wobei die Verengung der Atemwege mit einer akuten Kondition von Asthma bronchiale oder Status asthmaticus assoziiert ist.

5. Verwendung nach einem der vorhergehenden Ansprüche, wobei es sich bei dem Medikament um ein inhalierbares Medikament handelt.

6. Verwendung nach einem der vorhergehenden Ansprüche, wobei sich die Herstellung auf ein Medikament für die aufeinander folgende Verabreichung des Stickoxids und des COX-Inhibitors in beliebiger Reihenfolge bezieht.

7. Verwendung nach einem der Ansprüche 1-5, wobei das Medikament in der Form einer Zusammensetzung vorliegt, welche das Stickoxid und den Cyclooxigenaseinhibitor zu deren gleichzeitiger Verabreichung aufweist.

8. Verwendung nach einem der vorhergehenden Ansprüche, wobei der Cyclooxigenaseinhibitor aus der Gruppe ausgewählt ist, die aus Diclofenac, Aceclofenac, Nabumeton, Meloxicam, Meclofenamic, Nimesulid, Paracetamol, Rofecoxib, Celecoxib, DuP 697, GR 32191, Flosulid, NS 398, L-745,337, DFU, HN-56249, JTE-552, Aspirin, Indometacin und Ibuprofen oder Säureadditionssalzen davon besteht.

9. Verwendung nach einem der vorhergehenden Ansprüche, wobei NO in dem Medikament in einer Menge von 1-100 nmol/min vorhanden ist.

10. Verwendung nach einem der vorhergehenden Ansprüche, wobei die zu inhalierende Konzentration des NO im Bereich von 1-180 ppm, vorzugsweise 1-80 ppm, insbesondere 1-40 ppm liegt, wobei das NO in einem Trägergas oder einem Gasgemisch vorhanden ist.

11. Verwendung nach einem der vorhergehenden Ansprüche, wobei der Stickoxiddonor aus S-Nitroso-N-acetylpenicillamin, S-Nitrosocystein, Nitroprussid, Nitrosoguanidin, Glyceroltrinitrat, Isoamylnitrit, anorganischem Nitrit, Azid und Hydroxylamin ausgewählt ist.

## Revendications

1. Utilisation d'oxyde nitrique (NO) inhalable, sous forme d'oxyde nitrique gazeux ou d'un donneur d'oxyde nitrique, en combinaison avec un inhibiteur de cyclooxygénase, pour la préparation d'un médicament destiné au traitement de la vasoconstriction pulmonaire ou de la constriction des voies respiratoires chez un mammifère, notamment l'homme, dans le but de contrecarrer une hypo-réponse ou une non-réponse au traitement par l'oxyde nitrique gazeux ou un donneur d'oxyde nitrique seul, et/ou dans le but de contrecarrer une réponse formant rebond dans le cas de l'arrêt du traitement avec de l'oxyde nitrique gazeux ou un donneur d'oxyde nitrique seul.

2. Utilisation selon la revendication 1, dans laquelle ladite vasoconstriction pulmonaire ou ladite constriction des voies respiratoires est associée à un état clinique choisi dans le groupe consistant en la lésion traumatique, l'embolie graisseuse des poumons, l'acidose, le syndrome de détresse respiratoire de l'adulte, le mal aigu des montagnes, l'hypertension pulmonaire aiguë consécutive à une intervention chirurgicale cardiovasculaire et pulmonaire, une hypertension pulmonaire persistante du nouveau-né, un syndrome néonatal d'aspiration, une maladie des membranes hyalines, une thrombo-embolie pulmonaire aiguë, une maladie thrombo-embolique pulmonaire aiguë, un oedème pulmonaire aigu, des réactions héparine-protamine, une hypoxie et un asthme bronchique.

3. Utilisation selon la revendication 2, dans laquelle ladite constriction des voies respiratoires est associée à un asthme bronchique.

4. Utilisation selon la revendication 3, dans laquelle ladite constriction des voies respiratoires est associée à un état pathologique aigu d'asthme bronchique ou d'état de mal asthmatique.

5. Utilisation selon l'une quelconque des revendications précédentes, dans laquelle ledit médicament est un médicament inhalable.

6. Utilisation selon l'une quelconque des revendications précédentes, dans laquelle ladite préparation concerne un médicament pour administration successive, dans un ordre quelconque, dudit oxyde nitrique et dudit inhibiteur de COX.

7. Utilisation selon l'une quelconque des revendications 1 à 5, dans laquelle ledit médicament se présente sous forme d'une composition comprenant ledit oxyde nitrique et ledit inhibiteur de cyclooxygénase, pour leur administration simultanée.

8. Utilisation selon l'une quelconque des revendications précédentes, dans laquelle ledit inhibiteur de cyclooxygénase est choisi dans le groupe consistant en le diclofénac ; l'acéclofénac ; la nabumétone ; le méloxicam ; le méclofénamic ; le nimésulide ; le paracétamol ; le rofécoxib, le célécoxib, le DuP 697 ; le GR 32191 ; le flosulide ; le NS 398 ; le L-745, 337 ; le DFU ; le HN-56249 ; le JTE-552 ; l'aspirine ; l'indométacine ; et l'ibuprofène ; ou leurs sels d'addition avec un acide.

9. Utilisation selon l'une quelconque des revendications précédentes, dans laquelle le NO est présent dans ledit médicament en une quantité de 1 à 100 nmol/min.

10. Utilisation selon l'une quelconque des revendications précédentes, dans laquelle la concentration du NO devant être inhalé est comprise dans la plage de 1 à 180 ppm, de préférence de 1 à 80 ppm, tout spécialement de 1 à 40 ppm, ledit NO étant présent dans un gaz support ou dans un mélange gazeux.

11. Utilisation selon l'une quelconque des revendications précédentes, dans laquelle ledit donneur d'acide nitrique est choisi parmi la S-nitroso-N-acétylpénicillamine, la S-nitrosocystéine, un nitroprussiate, la nitrosoguanidine, le trinitrate de glycérol, le nitrite d'isoamyle, un nitrite inorganique, un azoture et l'hydroxylamine.
